# EUROPEAN PATENT APPLICATION

(11) **EP 2 191 829 A2**
(43) Date of publication of application: **02.06.2010**
(21) Application number: 09176308.6
(22) Date of filing: 03.08.2007
(51) Int. Cl.: A61K 31/4196, A61P 5/10, A61P 5/14, A61P 5/18, A61P 5/48

(54) **Treatment of endocrine dysfunction using iron chelators**

(30) Priority: 04.08.2006 EP 06118491
(62) Divisional of application: 07786560.8
(71) Applicant: Novartis AG, 4056 Basel (CH)
(72) Inventor: Cabantchik, Ioav, 90836, Har Adar (IL); Nick, Hanspeter, 4202, Duggingen (CH)
(74) Representative: Gruber, Markus

(57) **Abstract**

The invention relates to the use of an iron chelator for the treatment or prevention of pathologies due to iron loading, e.g. related to a dysfunction, in particular a reduction or inhibition, of the secretory function of cells from endocrine glands, in the human or animal body.

## Description

The present invention relates to the use of an iron chelator for the treatment or prevention of pathologies due to iron loading, e.g. related to a dysfunction, in particular a reduction or inhibition, of the secretory function of cells from endocrine glands by accumulated iron, in the human or animal body.

### BACKGROUND OF THE INVENTION

The Endocrine system is a complex network of glands that acts in concert with the nervous system to control and coordinate the myriad chemical reactions associated with storage and release of energy, growth, maturation, reproduction, and behavior. The sheer complexity of this system makers it vulnerable to breakdown, a fact that is reflected in numerous and varied endocrine disorders. The endocrine system's influence on bodily functions is so profound that many body systems may be affected when any of these glands fails to operate properly.

The endocrine system includes the pituitary, thyroid, parathyroid, adrenals, testes, ovaries, pineal, and thymus glands and the islet cells of the pancreas. The endocrine glands function by releasing hormones or chemical messengers into the bloodstream. These hormones trigger reactions in specific tissues.

This network of glands is regulated by the hypothalamus -- the area at the base of the brain where the endocrine system meets the nervous system - and by the pituitary gland. Together, they generate chemical messages that stimulate the other glands to further activity. When endocrine disorders develop, they usually consist of either hypofunction, e.g. underactivity or hyperfunction, e.g. overactivity, of one or more glands. Occasionally, inflammation or development of tumors in a gland leads to trouble as well.

Endocrine hyperfunction and hypofunction may have their source in the hypothalamus, the pituitary, or the target gland itself. Chronic disorders are more common, and generally lead to hypofunction; however, inflammation can cause acute episodic malfunctions. Tumors more commonly occur in the glands themselves, but can appear in other areas of the body, such as the lungs or stomach, where they produce hormones that cause endocrine dysfunction.

Accumulated iron interferes with the secretory function, and in particular causes reduction or inhibition of the secretory function, of cells of the endocrine glands. Reduction or inhibition can result in various pathologies like hypopituitarism (delayed sexual maturation, short stature, failure to thrive), hypothyroidism, hypoparathyriodism, diabetes (especially type 2 diabetes mellitus), impaired glucose metabolism (IGM), conditions of impaired glucose tolerance (IGT), conditions of impaired fasting plasma glucose (IFG), and diseases, disorders or conditions related/associated to diabetes (particularly type 2 diabetes mellitus) IGM or IGT.

The diseases, disorders or conditions related/associated to diabetes, particularly type 2 diabetes mellitus, IGM or IGT, includes but are not limited to diabetic nephropathy, diabetic retinopathy and diabetic neuropathy, macular degeneration, coronary heart disease, myocardial infarction, diabetic cardiomyopathy, myocardial cell death, coronary artery diseases, peripheral arterial disease, stroke, limb ischemia, vascular restenosis, foot ulcerations, endothelial dysfunction, atherosclerosis, increased microvascular complications, excess cerebrovascular diseases, increased cardiovascular mortality and sudden death.

Hypopituitarism is characterized by growth retardation in children, sexual immaturity, and metabolic dysfunction. Hypopituitarism results from a deficiency of the hormones secreted by the anterior pituitary gland. Panhypopituitarism involves a partial or total failure of all this gland's hormones.
Symptoms usually develop gradually and may include impotence, the absence of menstrual periods, infertility, decreased sexual drive, and diabetes insipidus (a state of high urinary output unrelated to the more common diabetes mellitus). Other symptoms are hypothyroidism and adrenal insufficiency (Addison's disease).

Hypothyroidism occurs more frequently in women than in men and is diagnosed most often between the ages of 40 and 50. Hypothyroidism, essentially an underproduction of thyroid hormone, can be caused by an insufficiency of the hypothalamus, the pituitary, or the thyroid gland itself.

An underactive thyroid gland may be the result of surgery, inflammation, autoimmune conditions, or insufficient iodine in the diet. Congenital defects may also cause hypothyroidism, and the condition can be a side effect of certain drugs.

The early symptoms of hypothyroidism tend to be vague. They include short-term memory loss, fatigue, lethargy, unexplained weight gain, intolerance to cold, poor wound healing, and constipation. Later signs of hypothyroidism include increased mental instability; puffiness in the face and extremities; thin, dry hair; loss of libido; loss of appetite; hand tremors; and abdominal bloating. If left untreated, hypothyroidism may eventually lead to onset of a life-threatening coma.

Hypoparathyroidism results from diseased, injured, or congenitally defective parathyroid glands. Injury to the glands most often occurs during surgery involving nearby tissue. Hypoparathyroidism may also be caused by the prolonged, severe magnesium deficiency associated with alcoholism.
Hypoparathyroidism leads to low blood concentrations of calcium, which may cause neuromuscular excitability, including spasms and twitching of the face, hands, and feet; abdominal pain; hair loss; dry skin; and cataracts.

There is still a need for the treatment and/or prevention of pathologies related to a dysfunction, of the endocrine glands, in particular a reduction or inhibition of the secretory function of cells from endocrine glands, by accumulated iron, in the human or animal body.

Iron chelators have been widely described in the literature. According to the observed binding to iron, the iron chelators may be classified into bidentate, tridentate or hexadentate chelators,

Specific bidentate iron chelators comprise 1,2-dimethyl-3-hydroxypyridin-4-one (Deferiprone, DFP or Ferriprox) and 2-deoxy-2-(N-carbamoylmethyl-[N'-2'-methyl-3'-hydroxypyridin-4'-one])-D-glucopyranose (Feralex-G).

Specific tridentate iron chelators comprise pyridoxal isonicotinyl hydrazone (PIH), 4,5-dihydro-2-(2,4-dihydroxyphenyl)-4-methylthiazole-4-carboxylic acid (GT56-252), 4,5-dihydro-2-(3'-hydroxypyridin-2'-yl)-4-methylthiaxole-4-carboxylic acid (desferrithiocin or DFT) and 4-[3,5-bis(2-hydroxyphenyl)-[1,2,4]triazol-1-yl]benzoic acid (ICL-670). Substituted 3,5-diphenyl-1,2,4-triazoles in the free acid form, salts thereof and its crystalline forms are disclosed in the International Patent Publication WO 97/49395, which is hereby incorporated by reference. Similarly a particularly advantageous pharmaceutical preparation of such compounds in the form of dispersible tablets is disclosed in the International Patent Publication WO 2004/035026, which is also hereby incorporated by reference.

Specific hexadentate iron chelators comprise N,N'-bis(o-hydroxybenzyl)ethylenediamine-N,N'-diacetic acid (HBED), N-(5-C3-L (5 aminopentyl) hydroxycarbamoyl)-propionamido)pentyl)-3(5-(N-hydroxyacetoamido)-pentyl)carbamoyl)-proprionhydroxamic acid (deferoxamine, desferrioxamine or DFO) and hydroxymethyl-starch-bound deferoxamine (S-DFO). Further derivatives of DFO include aliphatic, aromatic, succinic, and methylsulphonic analogs of DFO and specifically, sulfonamide-deferoxamine, acetamide-deferoxamine, propylamide deferoxamine, butylamide-deferoxamine, benzoylamide-deferoxamine, succinamide-derferoxamine, and methylsulfonamide-deferoxamine.

A further class of iron chelators is the biomimetic class (Meijler, MM, et al. "Synthesis and Evaluation of Iron Chelators with Masked hydrophilic Moieties" J. Amer. Chem. Soc. 124:1266-1267 (2002), is hereby incorporated by reference in its entirety). These molecules are modified analogues of such naturally produced chelators as DFP and ferrichrome. The analogues allow attachment of lipophilic moieties (e.g., acetoxymethyl ester). The lipophilic moieties are then cleaved intracellularly by endogenous esterases, converting the chelators back into hydrophilic molecules which cannot leak out of the cell.

### DETAILED DESCRIPTION OF THE INVENTION

It has now surprisingly been found that the inhibition of the secretory functions of cells from endocrine glands by accumulated iron can substantially be reduced or prevented by an iron chelator. Pathologies associated with such inhibition are in particular hypopituitarism (delayed sexual maturation, short stature, failure to thrive) hypothyroidism, hypoparathyriodism, diabetes (especially type 2 diabetes mellitus), impaired glucose metabolism (IGM), conditions of impaired glucose tolerance (IGT), conditions of impaired fasting plasma glucose (IFG), and diseases, disorders or conditions related/associated to diabetes (particularly type 2 diabetes mellitus) IGM or IGT.

One aspect of the invention is thus the use of an iron chelator for the preparation of a pharmaceutically acceptable preparation for the treatment and/or prevention of pathologies related to a dysfunction of the endocrine glands, in particular a reduction or inhibition of the secretory function of cells from endocrine glands, by accumulated iron, in the human or animal body.

A further aspect of the invention is the use of an iron chelator for the prevention, delay of progression or treatment of diabetes (especially type 2 diabetes mellitus), impaired glucose metabolism (IGM), conditions of impaired glucose tolerance (IGT), conditions of impaired fasting plasma glucose (IFG), and diseases, disorders or conditions related/associated to diabetes (particularly type 2 diabetes mellitus) IGM or IGT.

A further aspect of the invention is the use of an iron chelator for the treatment or prevention of pathologies, related to a dysfunction, in particular a reduction or inhibition of the secretory function of cells from endocrine glands by accumulated iron, in the human or animal body.

A further aspect of the invention is a method for the treatment or prevention of pathologies, related to a dysfunction, in particular a reduction or inhibition, of the secretory function of cells from endocrine glands by accumulated iron, in the human or animal body whereby a therapeutically effective amount of an iron chelator is administered to the human or animal in need thereof.

Useful iron chelators are, in particular, the bidentate, tridentate and hexadentate iron chelators as described in detail above.
Compounds of formula I as described below, and Compound I, i.e. 4-[3,5-Bis-(2-hydroxyphenyl)-[1,2,4]-triazol-1-yl]benzoic acid having the following formula 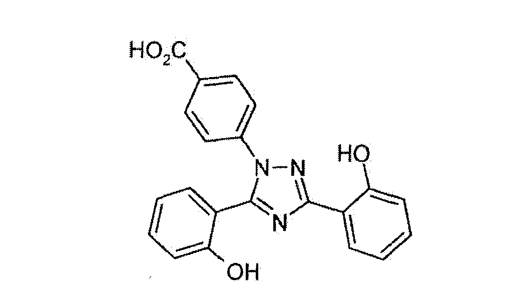 in the free acid form, salt thereof and its crystalline forms are disclosed in U.S. Patent No. 6,465,504 B1.

Compound I is an iron chelator that has been shown to be effective in the selective removal of iron in model systems and in humans, see e.g. Hershko C, et al. Blood. 2001, 97:1115-1122; Nisbet Brown E et al. Lancet. 2003, 361::1597-1602. However, Compound I was not known to be efficient in the treatment of hypopituitarism (delayed sexual maturation, short stature, failure to thrive), hypothyroidism, hypoparathyriodism, diabetes (especially type 2 diabetes inellitus), impaired glucose metabolism (IGM), conditions of impaired glucose tolerance (IGT), conditions of impaired fasting plasma glucose (IFG), and diseases, disorders or conditions related/associated to diabetes (particularly type 2 diabetes mellitus) IGM or IGT.

Particularly, there was a need to find a treatment of hypopituitarism (delayed sexual maturation, short stature, failure to thrive) hypothyroidism, hypoparathyriodism, diabetes and conditions related to diabetes due to iron overload of the endocrine organs.

Particularly useful as an iron chelator, in accordance with this invention, is a compound of the formula (I) 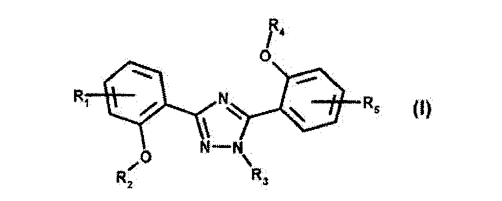 in which
R₁ and R₅ simultaneously or independently of one another are hydrogen, halogen, hydroxyl, lower alkyl, halo-lower alkyl, lower alkoxy, halo-lower alkoxy, carboxyl, carbamoyl, N-lower alkylcarbamoyl, N,N-di-lower alkylcarbamoyl or nitrile;
R₂ and R₄ simultaneously or independently of one another are hydrogen, unsubstituted or substituted lower alkanoyl or aroyl, or a radical which can be removed under physiological conditions;
R₃ is hydrogen, lower alkyl, hydroxy-lower alkyl, halo-lower alkyl, carboxy-lower alkyl, lower alkoxycarbonyl-lower alkyl, R₆R₇N-C(O)-lower alkyl, unsubstituted or substituted aryl or aryl-lower alkyl, or unsubstituted or substituted heteroaryl or heteroaralkyl;
R₆ and R₇ simultaneously or independently of one another are hydrogen, lower alkyl, hydroxy-lower alkyl, alkoxy-lower alkyl, hydroxyalkoxy-lower alkyl, amino-lower alkyl, N-lower alkylamino-lower alkyl, N,N-di-lower alkylamino-lower alkyl, N-(hydroxy-lower alkyl)amino-lower alkyl, N,N-di(hydroxy-lower alkyl)amino-lower alkyl or, together with the nitrogen atom to which they are bonded, form an azaalicyclic ring; or a pharmaceutically acceptable salt thereof.

Halogen is, for example, chlorine, bromine or fluorine, but can also be iodine.

The prefix "lower" designates a radical having not more than 7 and in particular not more than 4 carbon atoms.

Alkyl is straight-chain or branched. Per se, for example lower alkyl, or as a constituent of other groups, for example lower alkoxy, lower alkylamine, lower alkanoyl, lower alkylaminocarbonyl, it can be unsubstituted or substituted, for example by halogen, hydroxyl, lower alkoxy, trifluoromethyl, cyclo-lower alkyl, azaalicyclyl or phenyl, it is preferably unsubstituted or substituted by hydroxyl.

Lower alkyl is, for Example, *n*-propyl, isopropyl, *n*-butyl, isobutyl, *sec*-butyl, *tert*-butyl, *n*-pentyl, neopentyl, *n*-hexyl or *n*-heptyl; preferably methyl, ethyl and *n*-propyl. Halo-lower alkyl is lower alkyl substituted by halogen, preferably chlorine or fluorine, in particular by up to three chlorine or fluorine atoms.

Lower alkoxy is, for example, *n*-propoxy, isopropoxy, *n*-butoxy, isobutoxy, *sec*-butoxy, *tert-*butoxy, *n*-amyloxy, isoamyloxy, preferably methoxy and ethoxy. Halo-lower alkoxy is lower alkoxy substituted by halogen, preferably chlorine or fluorine, in particular by up to three chlorine or fluorine atoms.

Carbamoyl is the radical H₂N-C(O)-, N-lower alkylcarbamoyl is lower alkyl-HN-C(O)- and N,N-di-lower alkylcarbamoyl is di-lower alkyl-N-C(O)-.

Lower alkanoyl is HC(O)- and lower alkyl-C(O)- and is, for example, acetyl, propanoyl, butanoyl or pivaloyl.

Lower alkoxycarbonyl designates the radical lower alkyl-O-C(O)- and is, for example, *n*-propoxycarbonyl, isopropoxycarbonyl, *n*-butoxycarbonyl, isobutoxycarbonyl, *sec*-butoxycarbonyl, *tert*-butoxycarbonyl, *n*-amyloxycarbonyl, isoamyloxycarbonyl; preferably methoxycarbonyl and ethoxycarbonyl.

Aryl, per se, for example aryl, or as a constituent of other groups, for example aryl-lower alkyl or aroyl, is, for example, phenyl or naphthyl, which is substituted or unsubstituted. Aryl is preferably phenyl which is unsubstituted or substituted by one or more, in particular one or two, substituents, for example lower alkyl, lower alkoxy, hydroxyl, nitro, amino, halogen, trifluoromethyl, carboxyl, lower alkoxycarbonyl, amino, N-lower alkylamino, N,N-di-lower alkylamino, aminocarbonyl, lower alkylaminocarbonyl, di-lower alkylaminocarbonyl, heterocycloalkyl, heteroaryl or cyano. Primarily, aryl is unsubstituted phenyl or naphthyl, or phenyl which is substituted by lower alkyl, lower alkoxy, hydroxyl, halogen, carboxyl, lower alkoxycarbonyl, N,N-di-lower alkylamino or heterocycloalkylcarbonyl.

Aroyl is the radical aryl-C(O)- and is, for example, benzoyl, toluoyl, naphthoyl or *p*-methoxybenzoyl.

Aryl-lower alkyl is, for example, benzyl, *p*-chlorobenzyl, *o*-fluorobenzyl, phenylethyl, *p*-tolylmethyl; *p*-dimethylammobenzyl, *p*-diethylaminobenzyl, *p*-cyanobenzyl, *p*-pyrrolidinobenzyl.

Heterocycloalkyl designates a cycloalkyl radical having 3 to 8, in particular having from 5 to not more than 7, ring atoms, of which at least one is a heteroatom; oxygen, nitrogen and sulfur are preferred. Azaalicyclyl is a saturated cycloalkyl radical having 3-8, in particular 5-7, ring atoms, in which at least one of the ring atoms is a nitrogen atom. Azaalicyclyl can also contain further ring heteroatoms, e.g. oxygen, nitrogen or sulfur; it is, for example, piperidinyl, piperazinyl, morpholinyl or pyrrolidinyl. Azaalicyclyl radicals can be unsubstituted or substituted by halogen or lower alkyl. The azaalicyclyl radicals bonded via a ring nitrogen atom, which are preferred, are, as is known, designated as piperidino, piperazino, morpholino, pyrrolidino etc.

Heteroaryl per se, for example heteroaryl, or as a constituent of other substituents, for example heteroaryl-lower alkyl, is an aromatic radical having from 3 to not more than 7, in particular from 5 to not more than 7, ring atoms, in which at least one of the ring atoms is a heteroatom, e.g. pyrrolyl, imidazolyl, triazolyl, tetrazolyl, oxazolyl, thiazolyl, furanyl, thiophenyl, pyridyl, pyrazinyl, oxazinyl, thiazinyl, pyranyl or pyrimidinyl. Heteroaryl can be substituted or unsubstituted. Heteroaryl which is unsubstituted or substituted by one or more, in particular one or two, substituents, for example lower alkyl, halogen, trifluoromethyl, carboxyl or lower alkoxycarbonyl, is preferred.

Heteroaryl-lower alkyl designates a lower alkyl radical in which at least one of the hydrogen atoms, preferably on the terminal C atom, is replaced by a heteroaryl group if the alkyl chain contains two or more carbon atoms.

N-lower alkylamino is, for example, *n*-propylamino, *n*-butylamino, *i*-propylamino, *i*-butylamino, hydroxyethylamino, preferably methylamino and ethylamino. In N,N-di-lower alkylamino, the alkyl substituents can be identical or different. Thus N,N-di-lower alkylamino is, for example, NN-dimethylamino, N,N-diethylamino, N,N-methylethylamino, N-methyl-N-morpholinoethylamino, N-methyl-N-hydroxyethylamino, N-methyl-N-benzylamino.

Salts of compounds of the formula (I) are pharmaceutically acceptable salts, especially salts with bases, such as appropriate alkali metal or alkaline earth metal salts, e.g. sodium, potassium or magnesium salts, pharmaceutically acceptable transition metal salts such as zinc salts, or salts with organic amines, such as cyclic gamines, such as mono-, di- or tri-lower alkylamines, such as hydroxy-lower alkylamines, e.g. mono-, di- or trihydroxy-lower alkylamines, hydroxy-lower alkyl-lower alkylamines or polyhydroxy-lower alkylamines.
Cyclic amines are, for example, morpholine, thiomorpholine, piperidine or pyrrolidine. Suitable mono-lower alkylamines are, for example, ethyl- and *tert*-butylamine; di-lower alkylamines are, for example, diethyl- and diisopropylamine; and tri-lower alkylamines are, for example, trimethyl- and triethylamine. Appropriate hydroxy-lower alkylamines are, for example, mono-, di- and triethanolamine; hydroxy-lower alkyl-lower alkylamines are, for example, N,N-dimethylamino- and N,N-diethylaminoethanol; a suitable polyhydroxy-lower alkylamine is, for example, glucosamine. In other cases it is also possible to form acid addition salts, for example with strong inorganic acids, such as mineral acids, e.g. sulfuric acid, a phosphoric acid or a hydrohalic acid, with strong organic carboxylic acids, such as lower alkanecarboxylic acids, e.g. acetic acid, such as saturated or unsaturated dicarboxylic acids, e.g. malonic, maleic or fumaric acid, or such as hydroxycarboxylic acids, e.g. tartaric or citric acid, or with sulfonic acids, such as lower alkane- or substituted or unsubstituted benzenesulfonic acids, e.g. methane- or *p*-toluenesulfoniceacid. Compounds of the formula (I) having an acidic group, e.g. carboxyl, and a basic group, e.g. amino, can also be present in the form of internal salts, i.e. in zwitterionic form, or a part of the molecule can be present as an internal salt, and another part as a normal salt.

Preferably, the invention relates to above described use comprising at least one compound of the formula (I), in which
R₁ and R₅ simultaneously or independently of one another are hydrogen, halogen, hydroxyl, lower alkyl, halo-lower alkyl, lower alkoxy or halo-lower alkoxy; R₂ and R₄ simultaneously or independently of one another are hydrogen or a radical which can be removed under physiological conditions; R₃ is lower alkyl, hydroxy-lower alkyl, carboxy-lower alkyl, lower alkoxycarbonyl-lower alkyl, R₆R₇N-C(O)-lower alkyl, substituted aryl, aryl-lower alkyl, substituted by N-lower alkylamino, N,N-di-lower alkylamino or pyrrolidino, or unsubstituted or substituted heteroaryl or heteroaralkyl; R₆ and R₇ simultaneously or independently of one another are hydrogen, lower alkyl, hydroxy-lower alkyl, alkoxy-lower alkyl, hydroxyalkoxy-lower alkyl, amino-lower alkyl, N-lower alkylamino-lower alkyl, N,N-di-lower alkylamino-lower alkyl, N-(hydroxy-lower alkyl)amino-lower alkyl, N,N-di(hydroxy-lower alkyl)amino-lower alkyl or, together with the nitrogen atom to which they are bonded, form an azaalicyclic ring; and salts thereof; and at least one pharmaceutically acceptable carrier, and to methods for their preparation.

In one embodiment of the invention, the compound of formula (I) is 4-[3,5-bis(2-hydroxyphenyl)-[1,2,4]triazol-1-yl]benzoic acid or a pharmaceutically acceptable salt,

Suitable pharmaceutical preparations of the iron chelators, in particular of a compound according to formula (I), are those for enteral, in particular oral, and furthermore rectal, administration and those for parenteral administration to warm-blooded animals, especially to man, the pharmacological active ingredient being contained on its own or together with customary pharmaceutical adjuncts. The pharmaceutical preparations contain (in percentages by weight), for example, from approximately 0.001 % to 100%, preferably from approximately 0.1% to approximately 100%, of the active ingredient.

Oral formulations of 4-[3,5-bis(2-hydroxyphenyl)-[1,2,4]triazal-1-yl]benzoic acid or a pharmaceutically acceptable salt thereof are disclosed in the following International Patent Application publication WO97/49395 and WO 2004/035026, and said formulations are hereby incorporated by reference.

The dosage of the iron chelator, in particular of a compound of formula (I), can depend on various factors, such as activity and duration of action of the active ingredient, severity of the illness to be treated or its symptoms, manner of administration, warm-blooded animal species, sex, age, weight and/or individual condition of the warm-blooded animal. As an example, the doses to be administered daily in the case of oral administration of a compound of formula (I) are between 10 and approximately 120 mg/kg, in particular 20 and approximately 80 mg/kg, and for a warm-blooded animal having a body weight of approximately 40 kg, preferably between approximately 400mg and approximately 4,800 mg, in particular approximately 800 mg to 3,200 mg, which is expediently divided into 2 to 12 individual doses.

The present invention pertains to the use of an iron chelator for the preparation of a pharmaceutically acceptable preparation for the treatment or prevention of pathologies, related to a dysfunction, in particular a reduction or inhibition, of the secretory function of cells from endocrine glands by accumulated iron, in the human or animal body.

In one embodiment the invention pertains to the use of an iron chelator for the treatment or prevention of pathologies; related to a dysfunction, in particular a reduction or inhibition of the secretory function of cells from endocrine glands by accumulated iron, in the human or animal body.

The present invention further pertains to a method for the treatment or prevention of pathologies, related to a dysfunction, in particular a reduction or inhibition of the secretory function of cells from endocrine glands by accumulated iron, in the human or animal body whereby a therapeutically effective amount of an iron chelator is administered to the human or animal in need thereof, preferably whereby the iron chelator is a bidentate, tridentate or hexadentate iron chelator.

According the present invention, for the use or method as described above, the iron chelator is a compound of the formula (I) 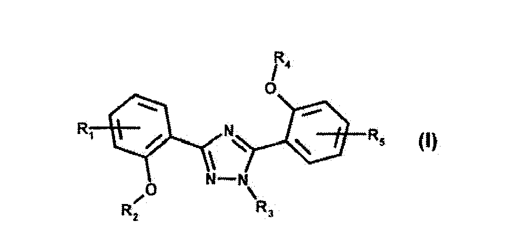 in which
R₁ and R₅ simultaneously or independently of one another are hydrogen, halogen, hydroxyl, lower alkyl, halo-lower alkyl, lower alkoxy, halo-lower alkoxy, carboxyl, carbamoyl, N-lower alkylcarbamoyl, N,N-di-lower alkylcarbamoyl or nitrile;
R₂ and R₄ simultaneously or independently of one another are hydrogen, unsubstituted or substituted lower alkanoyl or aroyl, or a radical which can be removed under physiological conditions;
R₃ is hydrogen, lower alkyl, hydroxy-lower alkyl, halo-lower alkyl, carboxy-lower alkyl, lower alkoxycarbonyl-lower alkyl, R₆R₇N-C(O)-lower alkyl, unsubstituted or substituted aryl or aryl-lower alkyl, or unsubstituted or substituted heteroaryl or heteroaralkyl;
R₆ and R₇ simultaneously or independently of one another are hydrogen, lower alkyl, hydroxy-lower alkyl, alkoxy-lower alkyl, hydroxyalkoxy-lower alkyl, amino-lower alkyl, N-lower alkylamino-lower alkyl, N,N-di-lower alkylamino-lower alkyl, N-(hydroxy-lower alkyl)amino-lower alkyl, N,N-di(hydroxy-lower alkyl)amino-lower alkyl or, together with the nitrogen atom to which they are bonded, form an azaalicyclic ring; or a pharmaceutically acceptable salt thereof.

According to the present invention, for the use or method as described above, the iron chelator is selected from the group consisting of 1,2-dimethyl-3-hydroxypyridin-4-one (Deferiprone, DFP or Ferriprox), 2-deoxy-2-(N-carbamoylmethyl-[N'-2'-methyl-3'-hydroxypyridin-4'-one])-D-glucopyranose (Feralex-G), pyridoxal isonicotinyl hydrazone (PIH), 4,5-dihydro-2-(2,4-dihydroxyphenyl)-4-methylthiazole-4-carboxylic acid (GT56-252), 4,5-dihydro-2-(3'-hydroxypyridin-2'-yl)-4-methylthiazole-4-carboxylic acid (desferrithiocin or DFT), 4-[3,5-bis(2-hydroxyphenyl)-[1,2,4]triazol-1-yl]benzoic acid (ICL-670), N,N'-bis(o-hydroxybenzyl)ethylenediamine-N,N'-diacetic acid (HBED), N-(5-C3-L (5 aminopentyl) hydroxycarbamoyl)-propionamido)pentyl)-3(5-(N-hydroxyacetoamido)-pentyl)carbamoyl)-proprionhydroxamic acid (deferoxamine, desferrioxamine or DFO), hydroxymethyl-starch-bound deferoxamine (S-DFO), sulfonamide-deferoxamine, acetamide-deferoxamine, propylamide deferoxamine, butylamide-deferoxamine, benzoylamide-deferoxamine, succinamide-derferoxamine, methylsulfonamide-deferoxamine and modified analogues of such naturally produced chelators as DFO and ferrichrome, preferably, the iron chelator is 4-[3,5-bis(2-hydroxyphenyl)-[1,2,4]triazol-1-yl]benzoic acid or a pharmaceutically acceptable salt thereof.

In a further aspect of the present invention, the pathologies as mentioned in the above embodiments of the inventions are hypopituitarism (delayed sexual maturation, short stature, failure to thrive), hypothyroidism, hypoparathyriodism, diabetes (especially type 2 diabetes mellitus), impaired glucose metabolism (IGM), conditions of impaired glucose tolerance (IGT), conditions of impaired fasting plasma glucose (IFG), and diseases, disorders or conditions related/associated to diabetes (particularly type 2 diabetes mellitus) IGM or IGT.

The following examples illustrate the invention.

### Example 1:

Introduction: Targeted disruption of the hemojuvelin gene in mice (HJV-/-) has recently been reported to cause markedly increased iron deposition, *inter alia,* in the pancreas (Niederkofler et al. 2005, Huang et al. 2005). The spontaneous iron loading and the effect of 4-[3,5-bis(2-hydroxyphenyl)-[1,2,4]triazol-1-yl]benzoic acid (Compound I) has been examined upon the removal of iron in HJV-/- mice with iron overload.

Methods: Inductively-coupled Plasma Optical Emission Spectrometry (ICP-OES, measurement of elemental iron) determination in spleen and pancreas were performed at week 8 (study start), 14, 24 and 28 in HJV -/- and wild type (wt) mice (n=6-8). Starting at week 20 three groups of HJV-/- animals received daily 0 (vehicle), 30 or 100 mg/kg of 4-[3,5-bis(2-hydroxyphenyl)-[1,2,4]triaxol-1-yl]benzoic acid, control wt-mice remained untreated. Iron loading was observed up to 28 weeks, including vehicle group, and the effects of 4-[3,5-bis(2-hydroxyphenyl)-[1,2,4]triazol-1-yl]benzoic acid were assessed by comparing the 28-week groups. MRI R2* measurements were performed using a 4.7T MR imager.

Results: Pancreas showed the most extreme difference in iron load (34-fold). Visual inspection of the time courses of iron loading between week 8 and 28 suggest a delayed loading of the pancreas.

Pancreas showed a clear trend to lower levels at the 100 mg/kg dose of 4-[3,5-bis(2-hydroxyphenyl)-[1,2,4]triazol-1-yl]benzoic acid.

| | |
|---|---|
| Treatment | pancreas iron ± SEM in mg/g dry weight |
| vehicle (n=7) | 6.07 ±1.04 |
| Compound 130 mg/kg (n=7) | 4.72 ± 0.65 |
| Compound 1100 mg/kg (n=6) | 3.54 ± 0.55 |
| n = number of animals; SEM = standard error of the mean | |

Conclusion: Compound I, i.e. 4-[3,5-bis(2-hydroxyphenyl)-[1,2,4]triazol-7-yl]benzoic acid, showed a trend to improvement of pancreatic iron load within 8 weeks of treatment.

### Example 2:

Exposure of pancreatic Min6 and pituitary Att20 cells (both highly active in endocytotic activity) shows that their exposure to labile iron, acutely or chronically, leads to major intracellular iron accumulation in organelles (endosomes, mitochondria, cytosol) and increased ROS formation when redox-prompting. Among the functions affected by metal-evoked ROS are cell permselectivity (calcein leakage), mitochondrial membrane potential (JC1 test), electron transport activity (Alamar Blue) and cell viability (calcein-propidiuin iodide). It can be observed that administration of deferasirox at therapeutically achievable doses (30-100 µM):
a. largely prevents labile iron from raising in cells if present in the iron loading medium and
b. reduces iron-evoked cell damage and increases cell viability if incubated with cells prior to or post iron-loading (acutely or chronically).

The studies indicate that deferasirox has both preventive and corrective potential against iron-evoked damage in iron-loaded endocrine and cardiac cells.

### ROS=reactive oxygen species

### Example 3: Cytoprotective effect of 4-[3,5-bis(2-hydroxyphenyl)-[1,2,4]triazol-1-yl]benzoic acid (Compound I) on At20 endocrine cells susceptible to iron-evoked oxidative damage

A major cause of biological damage associated with tissue iron accumulation is the cellular acquisition of circulating labile plasma iron (LPI) by endocytic mechanisms and ensuing metal-catalyzed oxidations. Endocrine cells are presumed to have endocytic abilities and/or high susceptibility to the formation of reactive oxygen intermediates (ROIs), particularly those generated in the presence of labile iron. As these properties might be implicated in the early onset of endocrine dysfunctions observed in transfusional hemosiderosis, a major goal of iron chelation therapy is to prevent iron ingress into endocrine cells and ensuing toxicity and/or removal of cell accumulated iron. Such properties of chelators have been afforded in cardiac cells (Glickstein et al, 2006, Blood In press) by treating LPI-carrying plasma with pharmacological attainable concentrations of orally active iron chelators in clinical practice. The latter also evoked neutralization of metal catalyzed ROS formation, by iron chelation and extrusion from cells and thereby supported cardiac functional restoration. It has been explored here whether addition of 4-[3,5-bis(2-hydroxyphenyl)-[1,2,4]triazol-1-yl]benzoic acid could rescue acutely- iron -overloaded At20 pituitary cells from iron-toxicity, using fluorescence-plate reader and microscopy-imaging for live monitoring of cell functions and assessing toxicity in terms of: 1, cell integrity (calcein cell retention assay) 2. metabolic activities (mitochondrial Alamar-Blue assay) and 3. intracellular (ACTH) hormone levels. The studies indicate that in cell culture conditions and at pharmacological chelator concentrations, 4-[3,5-bis(2-hydroxyphenyl)-[1,2,4]triazol-1-yl]benzoic acid can preserve both At20 endocrine cell viability and functional properties that were compromised by iron-catalyzed formation of ROIs.

The insulin secretion enhancing properties of the combination according to the present invention may be determined by following the methodology as disclosed, for example, in the publication of T.Ikenoue et al. Biol.Pharm.Bull. 29(4), 354-359 (1997).
The simultaneous evaluation of the cardiovascular actions and of the glucose utilization effects of the agents given alone or in combination can be performed using models such as the Zucker fatty rat as described in the publication of Nawano et al., Metabolism 48: 1248-1235, 1999. The corresponding subject matter of these references is herewith incorporated by reference in this specification.

### Example 4: Effect of 4-[3,5-bis(2-hydroxyphenyl)-[1,2,4]triazol-1-yl]benzoic acid (Compound I) on Type 2 diabetes/metabolic disease

Diet-induced obese mice are used for the study at 21-23 weeks of age. On the first day of the study, animals are fasted at 7:30 a.m. Body weight measurement and basal blood sample collection are conducted at 10:30 a.m. Plasma glucose values are then determined. Animals were assigned into 4 groups (n = 10/groups) with the plasma glucose values and body weights matched between the 4 groups. At 12 pm, the animals are dosed orally with vehicle (water) or the Compound I at 30 mg/kg or 100 m/kg respectively. At 1:00 p.m. a blood sample (at 0 min) is taken followed by an oral glucose tolerance test (OGTT) at 1 g/kg (20% glucose in water) at a dose volume of 5 ml/kg. Blood samples are collected at 30, 60 and 120 min following the glucose administration. The animals are refed after the OGTT. The animals are administered a daily dose of vehicle or the compound 12:00 p.m. each day for a total of 15 days. Daily body weight and food intake measurements are performed during the study. Two additional OGTTs are performed during the study on the days 7 and 14, following the protocol described above for the OGTT on day 1. Animals treated with Compound I (30 mg/kg or 100 m/kg respectively) can show an improvement in glucose tolerance as compared to the control animals, as measured by the area under the curve during an OGTT. The magnitude of improvement in the OGTT increases in a time-dependent manner from day 7 to day 14. On the last day of the study (day 15), mice are fasted at 7:30 a.m. and dosed with vehicle or compound at 10:30 a.m.Tail blood samples are taken at 12:30 p.m. Animals are then euthanized with carbon dioxide. Terminal blood samples are collected via cardiac puncture for blood chemistry analysis.

### Blood collection and analyses

Blood samples are taken during the study via tail bleeding. Plasma glucose concentrations are determined using a glucose meter (Ascensia Elite, Bayer Corp., Mishawaka, IN). Blood samples were collected in tubes (Microvette CB300, Aktiengesellschaft & Co., Numbrecht, Germany) which contain lithium heparin to prevent blood clotting. Prior to each blood sample collection, 1µl of 1:10 diluted protease inhibitor cocktail (Sigma, St. Louis, MO) is added to the sample tubes. After blood sample collection, the tubes are kept on ice before being centrifuged. The plasma portion of the blood samples is obtained by centrifugation at 10,000 x g for 10 min at 4 °C and then stored at -80°C. Plasma insulin and glucagons levels are determined by Luminex assays using Mouse Endocrine Linco*plex* kit (Linco Research, Inc., St. Charles, MO). Animals treated with Compound I can show a lowering in plasma insulin levels as compared to the control animals. Plasma triglyceride, fatty acid and total cholesterol levels are determined using a fluorescent assay based on Amplex Red kit (Molecular Probes, Eugene, OR). Blood chemistry analysis is performed using an automated dry chemistry system (SPOTCHEM EZ Analyzer, Heska, Fort Collins, CO). Effects on cardiovascular diseases can also be assessed by further analysis on treated animals.

### Example 5: Effect of 4-[3,5-bis(2-hydroxyphenyl)-[1,2,4]triazol-1-yl]benzoic acid (Compound I) on IGM patients: Treatment, prevention of type 2 diabetes and cardiovascular diseases.

Favorable effects can be verified that conform that Compound I can restore early phase insulin secretion and reduce post-prandial glucose levels in subjects with IGM, or can prevent or delay the onset of Type 2 diabetes and cardiovascular diseases in subjects with IGM. A multi-center, double-blind, parallel group, randomized study can be conducted in subjects with IGM in order to evaluate the incidence of confirmed hypoglycemia and the effects on prandial glucose associated with the administration of Compound I, or placebo before each main meal during 8 weeks of treatment. Subjects are selected on the basis of a 2-hour plasma glucose value after a 75 g oral glucose tolerance test (OGTT) and patients essentially meeting the following additional inclusion criteria are included in the study:
- two-hour glycemia post-OGTT between 7.8 to 11.1 mmol/L (one OGTT to be performed during the year before entering the study, the second to be performed within two weeks prior entering the study);
- FPG < 7 mmol/L;
- patients are to have a body mass index (BMI) between 20-32 kg/m2;
- patients are to maintain prior diet during the full course of study;
- males, non-fertile females, females of child-bearing potential using a medically approved birth control method are included;
- the use of other antidiabetics during the trial is not permitted.

Corresponding dosages of Compound I are administered daily depending on the number of main meals (breakfast, lunch, snack, dinner). The first dose is to be given with the first main meal (standardized meal i.e. 55% carbohydrates, 25% fat and 20% protein). Visits are scheduled to be performed at weeks 0, 2, 4 and 8 and the patients are to be fasted for at least 7 hours. All blood samples for laboratory evaluations are drawn between 7.00 a.m. and 10.00 a.m. HbA1c is to be measured at baseline and after 8 weeks of treatment (fasting glucose and fructosamine). Samples of blood are to be drawn at 10, 20, 20, 60, 120, and 180 minutes after drug administration (time 0) and the glucose and insulin levels to be measured. At weeks 0 and 8 visits, patients complete a standard meal challenge containing approximately 500 kcal and measurements of insulin and glucose will be performed.

The findings from analyses of all obtained data in such a study can reveal that 2 hour prandial glucose levels, HBA1c and fructosamine levels can be reduced, that early phase insulin secretion can be restored, and that Compound I can prevent or delay the progression to type 2 diabetes mellitus. With longer treatment and follow-up, the preventive and reduction effect on conditions and diseases associated with IGM e.g. cardiovascular diseases can be evaluated, i.e. prevention or delay of progression to overt diabetes mellitus type 2; or prevention, reduction or delay in onset of a cardiovascular condition or disease associated with IGT preferably selected from the group consisting of increased microvascular complications; increased cardiovascular morbidity; excess cerebrovascular diseases; increased cardiovascular mortality and sudden death

This type of study in individuals with IGM and particularly IFG and IGT differs from those in diabetics since the subjects have normal FPG and are non-diabetics or pre-diabetics.

## Claims

1. Use of an iron chelator for the preparation of a pharmaceutically acceptable preparation for the treatment or prevention of pathologies due to iron loading, related to a dysfunction, in particular a reduction or inhibition, of the secretory function of cells from endocrine glands by accumulated iron, in the human or animal body.

2. Use of an iron chelator for the treatment or prevention of pathologies due to iron loading, related to a dysfunction, in particular a reduction or inhibition of the secretory function of cells from endocrine glands by accumulated iron, in the human or animal body.

3. Method for the treatment or prevention of pathologies, related to a dysfunction, in particular a reduction or inhibition of the secretory function of cells from endocrine glands by accumulated iron, in the human or animal body whereby a therapeutically effective amount of an iron chelator is administered to the human or animal in need thereof.

4. Use or method according to any one of claims 1 to 3, whereby the iron chelator is a bidentate, tridentate or hexadentate iron chelator.

5. Use or method according to any one of claims 1 to 4, whereby the iron chelator is a compound of the formula (I) 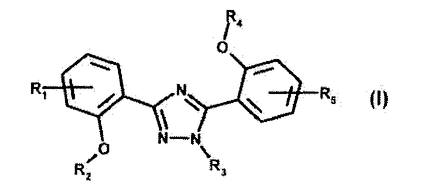 in which
R₁ and R₅ simultaneously or independently of one another are hydrogen, halogen, hydroxyl, lower alkyl, halo-lower alkyl, lower alkoxy, halo-lower alkoxy, carboxyl, carbamoyl, N-lower alkylcarbamoyl, N,N-di-lower alkylcarbamoyl or nitrile;
R₂ and R₄ simultaneously or independently of one another are hydrogen, unsubstituted or substituted lower alkanoyl or aroyl, or a radical which can be removed under physiological conditions;
R₃ is hydrogen, lower alkyl, hydroxy-lower alkyl, halo-lower alkyl, carboxy-lower alkyl, lower alkoxycarbonyl-lower alkyl, R₆R₇N-C(O)-lower alkyl, unsubstituted or substituted aryl or aryl-lower alkyl, or unsubstituted or substituted heteroaryl or heteroaralkyl;
R₆ and R₇ simultaneously or independently of one another are hydrogen, lower alkyl, hydroxy-lower alkyl, alkoxy-lower alkyl, hydroxyalkoxy-lower alkyl, amino-lower alkyl, N-lower alkylamino-lower alkyl, N,N-di-lower alkylamino-lower alkyl, N-(hydroxy-lower alkyl)amino-lower alkyl, N,N-di(hydroxy-lower alkyl)amino-lower alkyl or, together with the nitrogen atom to which they are bonded, form an azaalicyclic ring; or a pharmaceutically acceptable salt thereof.

6. Use or method according to any one of claims 1 to 4, whereby the iron chelator is selected from the group consisting of 1,2-dimethyl-3-hydroxypyridin-4-one (Deferiprone, DFP or Ferriprox), 2-deoxy-2-(N-carbamoylmethyl-[N'-2'-methyl-3'-hydroxypyridin-4'-one])-D-glucopyranose (Feralex-G), pyridoxal isonicotinyl hydrazone (PIH), 4,5-dihydro-2-(2,4-dihydroxyphenyl)-4-methylthiazole-4-carboxylic acid (GT56- 252), 4,5-dihydro-2-(3'-hydroxypyridin-2'-yl)-4-methylthiazole-4-carboxylic acid (desferrithiocin or DFT), 4-[3,5-bis(2-hydroxyphenyl)-[1,2,4]triazol-1-yl]benzoic acid (ICL-670), N,N'-bis(o-hydroxybenzyl)ethylenediamine-N,N'-diacetic acid (HBED), N-(5-C3-L(5aminopentyl) hydroxycarbamoyl)-propionamido)pentyl)-3(5-(N-hydroxyacetoamido)-pentyl)carbamoyl)-proprionhydroxamic acid (deferoxamine, desferrioxamine or DFO), hydroxymethyl-starch-bound deferoxamine (S-DFO), sulfonamide-deferoxamine, acetamide-deferoxamine, propylamide deferoxamine, butylamide-deferoxamine, benzoylamide-deferoxamine, succinamide-derferoxamine, methylsulfonamide-deferoxamine and modified analogues of such naturally produced chelators as DFO and ferrichrome.

7. Use or method according to any one of claims 1 to 6, whereby the iron chelator is 4-[3,5-bis(2-hydroxyphenyl)-[1,2,4]triazol-1-yl]benazoic acid or a pharmaceutical acceptable salt thereof.

8. Use or method according to any one of claims 1 to 7, whereby the pathology is hypopituitarism (delayed sexual maturation, short stature, failure to thrive).

9. Use or method according to any one of claims 1 to 7, whereby the pathology is hypothyroidism.

10. Use or method according to any one of claims 1 to 7, whereby the pathology is hypoparathyriodism.

11. Use or method according to any one of claims 1 to 7, whereby the pathology is diabetes (especially type 2 diabetes mellitus), impaired glucose metabolism (IGM), conditions of impaired glucose tolerance (IGT), conditions of impaired fasting plasma glucose (IFG), and diseases, disorders or conditions related/associated to diabetes (particularly type 2 diabetes mellitus) IGM or IGT.

12. Use or method according to any one of claims 1 to 7, whereby the pathology is type 2 diabetes.
